# EUROPEAN PATENT SPECIFICATION

(11) **EP 3 263 543 B1**
(45) Date of publication and mention of the grant of the patent: **02.12.2026**
(21) Application number: 17180528.6
(22) Date of filing: 07.10.2011
(51) Int. Cl.: C07C 17/23, C07C 21/18, C07C 19/08, C07C 17/38

(54) **INTEGRATED PROCESS FOR THE MANUFACTURE OF FLUORINATED OLEFINS**
INTEGRIERTES VERFAHREN ZUR HERSTELLUNG FLUORIERTER OLEFINE
PROCÉDÉ INTÉGRÉ POUR LA FABRICATION D'OLÉFINES FLUORÉES

(30) Priority: 12.10.2010 US 392242 P; 09.09.2011 US 201113229016
(43) Date of publication of application: 03.01.2018
(60) Divisional application: 26192812.1 / 4 799 998
(62) Document number(s) of the earlier application(s) in accordance with Art. 76 EPC: 11833160.2 / 2 627 620
(73) Proprietor: Solstice Advanced Materials US, Inc., Morris Plains, NJ 07950 (US)
(72) Inventor: BEKTESEVIC, Selma, Morristown, NJ New Jersey 07962-2245 (US); TUNG, Hsueh Sung, Morristown, NJ New Jersey 07962-2245 (US); KOPKALLI, Haluk, Morristown, NJ New Jersey 07962-2245 (US)
(74) Representative: Stepney, Gregory John

(56) References cited:
- EP-A1- 2 100 867
- FR-A1- 2 940 968

## Description

### FIELD OF THE INVENTION

The present invention relates to processes for producing haloalkenes 2,3,3,3-tetrafluoropropene (HFO-1234yf) and/or 1,2,3,3,3-pentafluoropropene (HFO-1225ye).

### BACKGROUND OF THE INVENTION

Chlorine-containing compounds, such as chlorofluorocarbons (CFCs), have been employed as refrigerants, foam blowing agents, cleaning agents, solvents, heat transfer media, sterilants, aerosol propellants, dielectrics, fire extinguishing agents, and power cycle working fluids. Such chlorine-containing compounds have proven to be detrimental to the Earth's ozone layer. Many of the hydrofluorocarbons (HFCs), used as the substitutes of CFCs, have been found to contribute to global warming. For these reasons, there is a worldwide effort to develop new compounds that are more environmentally benign while at the same time being as effective, or more effective, from a performance standpoint.

Applicants have come to appreciate that 1,1,1,2,3-pentafluoropropene (HFO-1225ye) and 1,1,1,2-tetrafluoropropene (HFO-1234yf) are each useful in one or more of the above mentioned applications. Accordingly, compositions containing either or both fluorinated olefins are among the materials being developed for such use.

Methods for producing HFO-1234yf and HFO-1225ye are known. EP 2 100 867 A discloses a process for producing at least one fluorinated olefin, such as HFO-1234yf. In one example, it is known that hexafluoropropylene (HFP) can be hydrogenated to produce 1,1,1,2,3,3-hexafluoropropane (HFC-236ea). HFC-236ea is then used as a reactant in a dehydrogenation reaction to produce HFO-1225ye. It is further known that HFO-1225ye can be hydrogenated to produce 1,1,1,2,3-pentafluoropropane (HFC-245eb) and that HFC-245eb can then be dehydrofluorinated to produce HFO-1234yf.

U.S. Patent Application Publication No. 2009/0234165 further provides that HFO-1225ye and HFO-1234yf can be produced in a single facility. Most notably, it was realized that the hydrogenation of HFP can yield both HFC-236ea and HFC-245eb and that these two products can be simultaneously dehydrofluorinated to produce HFO-1225ye and HFO-1234yf, respectively. Processing conditions are taught to be adjustable, so as to favor the selective conversion of one hydrofluoroolefin over the other. Catalysts that may be used for such reactions were taught to include metal catalysts, even more preferably one or more transition metal-based catalysts (including in certain preferred embodiments transition metal halide catalysts), such as FeCl₃, chromiumoxyfluoride, Ni (including Ni mesh), NiCl₂, CrF₃, and mixtures thereof, supported or in bulk. Other catalysts include carbon-supported catalysts, antimony-based catalysts (such as SbCl₅), aluminum-based catalyst (such as AlF₃, Al₂O₃, and fluorinated Al₂O₃), palladium-based catalysts, platinum-based catalysts, rhodium-based catalysts and ruthenium-based catalysts, including combinations thereof.

Other examples of methods for the production of HFO-1225ye and HFO-1234yf are set forth in, at least, U.S. Pat. No. 7,560,602. FR 2 940 968 A discloses a process for the continuous or semi-continuous manufacture of a (hydro)fluoroolefin compound. This patent discloses a similar dehydrohalogenation process for producing 2,3,3,3-tetrafluoropropene (1234yf) and 1,1,1,2,3-pentafluoropropene (HFO-1225ye) by catalytic dehydrofluorination of 1,1,1,2,3-pentafluoropropane (245eb) and 1,1,1,2,3,3-hexafluoropropane (HFC-236ea), respectively. Preferred dehydrohalogenation catalysts include fluorinated chromium oxide catalysts, aluminum fluoride catalysts, ferric fluoride catalysts, mixtures of magnesium fluoride and aluminum fluoride catalysts, nickel-based catalysts, carbon based catalysts, and combinations thereof.

Alternative agents for such dehydrohalogenation reactions are also known. U.S. Patent Application Publication No. 20100029997, for example, teaches the production of hydroolefins (e.g. HFO-1234yf) by dehydrohalogenating HFC-245eb by contacting it to potassium hydroxide (KOH), sodium hydroxide (NaOH), Ca(OH)₂, CaO, and combinations thereof. While, in certain embodiments, dehydrohalogenation agents include KOH, alternative agents also include LiOH, Mg(OH)₂ and NaOH.

Applicants have come to appreciate that during continuous processing using such dehydrohalogenation agents (also referred to as "reagent"), the reaction proceeds until either the organic reactants or dehydrohalogenation agent is consumed. Upon completion of the reaction, the reactor must be dismantled to remove the salt and/or salt solutions. This, in turn creates higher operating costs and reduces productivity. Accordingly, more efficient process is desirable for removal of spent reagent and recycle of unused or regenerated reagent and/or unused organics during a continuous process.

Applicants assert that the instant invention addresses the foregoing needs.

### SUMMARY OF THE INVENTION

The instant invention relates, at least in part, to a method of increasing the cost efficiency for dehydrohalogenation production of a fluorinated olefin by recovering and recycling spent reagent. In one aspect, the present invention relates to dehydrohalogenating a fluorinated alkane (e.g. pentafluoropropanes and/or hexafluoropropanes) in the presence of a dehydrohalogenating agent to produce a fluorinated olefin (e.g. tetrafluoropropenes and/or pentafluoropropenes). The dehydrohalogenating agent is KOH, wherein additional dehydrohalogenating agents may include, but are not limited to, sodium hydroxide (NaOH), calcium hydroxide (Ca(OH)₂), lithium hydroxide (LiOH), magnesium hydroxide (Mg(OH)₂), calcium oxide (CaO), or combinations thereof. Removal of spent dehydrohalogenating agent from the reactor allows for facile separation of the organic and the dehydrohalogenating agent. This in turn leads to lower cost associated with design and operation of complicated and highly specialized separation equipment. Recycling the spent dehydrohalogenating agent leads to greater efficiency. Increased productivity is similarly observed if metal fluoride salts are also isolated from the product stream, converted back to the dehydrohalogenating agent's original form, and recycled to the reaction.

In embodiments where the second product haloolefin is HFO-1225ye and/or HFO-1234yf, for example, the foregoing reactions can be produced in a single facility having four unit operations. More specifically, hydrogenation of HFP with hydrogen is known to yield both HFC-236ea and HFC-245eb. Like HFC-236ea, HFC-245eb subsequently can be dehydrofluorinated to produce a desirable product. In particular, HFC-236ea can be dehydrofluorinated to produce HFO-1225ye and HFC-245eb can be dehydrofluorinated to produce HFO-1234yf. Thus, both HFO-1225ye and HFO-1234yf can be produced using a single set of four unit operations: hydrogenation of the starting material, separation of the desired intermediate, dehydrofluorination of the intermediate to produce the desired product, followed by another separation to isolate the desired product. For example, in a one system, HFP and H₂ are reacted in a hydrogenation reactor to form an intermediate product stream comprising HFC-236ea and/or HFC-245eb. The relative concentrations of HFC-236ea and HFC-245eb is dependant on reaction conditions in the hydrogenation reactor, such as pressure, temperature, and relative concentration of reactants in the reactor. If the desirable end product is HFO-1225ye, then processing conditions preferably favor the production of HFC-236ea. That is, the hydrogenation reactor is operated to produce an intermediate product stream rich in HFC-236ea which is subsequently separated from the intermediate process stream and fed into a dehydrofluorination reactor to form a final product stream comprising HFO-1225ye. This HFO-1225ye is then separated from the final product stream and recovered as a purified product. If the desirable end product is HFO-1234yf, then processing conditions preferably favor the production of HFC-245eb. That is, the hydrogenation reactor is operated to produce an intermediate product stream rich in HFC-245eb. This can be accomplished by operating the hydrogenation reactor under conditions favorable to convert the HFP into HFC-236ea and then converting the HFC-236ea into HFC-245eb. The HFC-245eb is then separated from the intermediate product stream and fed into a dehydrofluorination reactor to form a final product stream comprising HFO-1234yf. This HFO-1234yf is then separated from the final product stream and recovered as a purified product.

In addition, when HFO-1234yf is the desired product, HFO-1225ye can be introduced into the hydrogenation reactor at some point and then converted into HFC-245eb. The source of this HFO-1225ye can be either a separate feed stream and/or a recycle stream (i.e., recycling HFO-1225ye derived from HFC-236ea as noted above). The HFC-245eb is again separated from the intermediate product stream and fed into a dehydrofluorination reactor to form a final product stream comprising HFO-1234yf. This HFO-1234yf is then separated from the final product stream and recovered as a purified product.

With any of the foregoing, the dehydrofluorination steps (e.g. 245eb → 1234yf or 236ea → 1225ye) occurs in the presence of at least one dehydrohalogenating agent that is KOH, wherein additional dehydrohalogenating agents include, such as, but not limited to, sodium hydroxide (NaOH), calcium hydroxide (Ca(OH)₂), lithium hydroxide (LiOH), magnesium hydroxide (Mg(OH)₂), calcium oxide (CaO), or combinations thereof. The amount of reagent used, or mole ratio of reagent to organic, will vary depending on the particular parameters present in each embodiment as discussed above, or otherwise herein. In certain embodiments, the mol ratio of dehydrohalogenating agent to organic feeds (e.g. HFC-245eb and/or HFC-236ea) is from less than 1 to 3, preferably 1-1.5.

Dehydrohalogenation can be performed in any suitable reaction vessel or reactor. In certain non-limiting embodiments, the reactor includes one or a series of Continuously Stirred Tank Reactors (CSTR), where organic (e.g. HFC-236ea and/or HFC-245eb) and dehydrohalogenating agent are fed continuously into the reactor. From the reaction product stream, a stream of spent dehydrohalogenating agent may be withdrawn and purified on a continuous or intermittent basis.

The stream of spent dehydrohalogenating agent further includes one or more dissolved organics, such as, but not limited to HFC-236ea and/or HFC-245eb. The spent dehydrohalogenating agent may be purified from such organics using one or more known separation methods. Such methods include, but are not limited to, distillation and/or phase separation. The resulting dehydrohalogenating agent can then be, optionally, concentrated and recycled back to the dehydrohalogenation reaction independently, or otherwise with one or more organics, as provided herein.

The product stream also includes a salt by-product of the dehydrohalogenation reaction. The salt by-product may be isolated from the product stream using known techniques and converted back to the dehydrohalogenating agent using known methods. It may then be recycled to the dehydrohalogenation reaction independently or with one or more of the organics provided herein. By way of non-limiting example, one such salt by-product is potassium fluoride (KF), which is formed since potassium hydroxide is used as the dehydrohalogenation agent. KF may be converted to KOH by treatment with Ca(OH)₂ (calcium hydroxide) according to the reaction:

2KF + Ca(OH)₂ → 2KOH + CaF₂

The resulting KOH can be, optionally, concentrated and recycled back to the dehydrohalogenation reaction independently, or otherwise with one or more organics, as provided herein.

Additional embodiments and advantages of the instant invention will be readily apparent to one of skill in the art based on the disclosure provided herein.

### BRIEF DESCRIPTION OF THE DRAWINGS

FIG. 1 is a process flow diagram showing the production of fluoroolefins according to an embodiment of the invention.
FIG. 2 is a process flow diagram showing the hydrogenation unit operation according to an embodiment of the invention.
FIG. 3 is a process flow diagram showing the hydrogenation unit operation according to another embodiment of the invention.
FIG. 4 is a process flow diagram showing the first separation unit operation according to an embodiment of the invention.
FIG. 5 is a process flow diagram showing the first separation unit operation according to another embodiment of the invention.
FIG. 6 is a process flow diagram showing the dehydrofluorination unit operation according to an embodiment of the invention.
FIG. 7 is a process flow diagram showing the dehydrofluorination unit operation according to another embodiment of the invention.

### DETAILED DESCRIPTION OF THE INVENTION

The instant invention relates, at least in part, to a method increasing the cost efficiency for dehydrohalogenation production of a fluorinated olefin by recovering and recycling spent dehydrohalogenation agent. In one aspect, the present invention relates to dehydrohalogenating a fluorinated alkane (e.g. pentafluoropropane and/or hexafluoropropane) in the presence of a dehydrohalogenating agent to produce a fluorinated olefin (e.g. tetrafluoropropenes and/or pentafluoropropenes). Removal of spent dehydrohalogenating agent from the reactor reduces design and operation costs and recycling the spent and/or regenerated dehydrohalogenating agent leads to improved efficiency. The dehydrohalogenating agent is KOH, wherein additional dehydrohalogenating agents include, but are not limited to, sodium hydroxide (NaOH), calcium hydroxide (Ca(OH)₂), lithium hydroxide (LiOH), magnesium hydroxide (MgOH), calcium oxide (CaO) or combinations thereof. In further embodiments, the dehydrohalogenating agent is potassium hydroxide (KOH).

In any of the foregoing reactions, the step wherein the alkene is converted to an alkane is carried out under conditions effective to provide a olefin conversion of at least about 40%, more preferably at least about 55%, and even more preferably at least about 70%. In certain preferred embodiments the conversion is at least about 90%, and more preferably about 99%. Further in certain preferred embodiments, the conversion of the alkene to produce an alkane is conducted under conditions effective to provide a selectivity of at least about 60%, more preferably at least about 80%, and more preferably at least about 90%, and even more preferably about 100%.

In any of the foregoing reactions, the step wherein the alkane is converted to a fluorinated olefin is carried out under conditions effective to provide a conversion of at least about 40%, more preferably at least about 55%, and even more preferably at least about 70%. In certain preferred embodiments the conversion is at least about 90%, and more preferably about 95%. Further in certain preferred embodiments, the conversion of the alkane to produce the fluorinated olefin is conducted under conditions effective to provide a selectivity of at least about 60%, more preferably at least about 80%, and more preferably at least about 90%, and even more preferably about 98%.

### THE HYDROGENATION STEP

Although it is contemplated that the hydrogenation or reduction step may be conducted in batch operation, it is preferred that the hydrogenation reaction is carried out as a substantially continuous operation. While it is contemplated that the hydrogenation reaction may be conducted in a single reaction vessel, this reaction step may comprise two or more reactors or reaction stages in parallel, in series, or both, or any combination of reactor designs. In addition, it is contemplated that the reaction step may include one or more feed preheating steps or stages, depending on the particulars of each application.

While it is possible that the reaction may involve in certain embodiments a liquid phase reaction, it is contemplated that in certain embodiments the hydrogenation reaction comprises at least one vapor phase reaction stage.

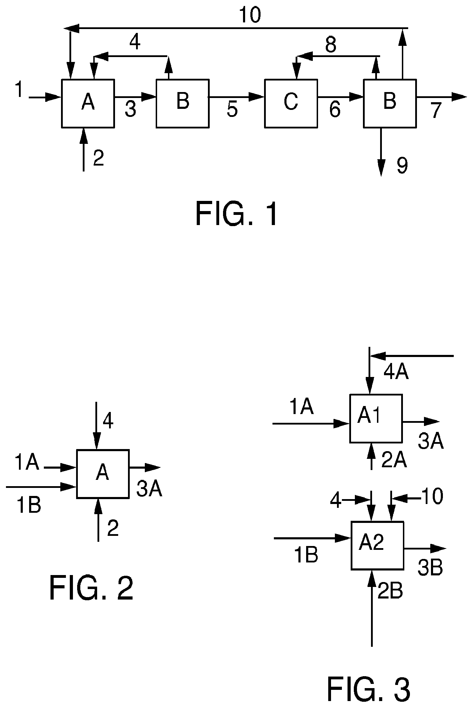

In one embodiment of the present invention, as illustrated in Figure 2, the hydrogenation step comprises a reaction step A having associated therewith at least a first flow path or feed stream 1A, at least a second flow path or feed stream 1B and at least a third flow path or feed stream 2, with each flow path being independently operable. In such embodiments, it is preferred that the first flow path or feed stream 1A comprises HFP and preferably substantially all of the HFP being fed to the reaction step A, the second path or feed stream 1B comprises HFO-1225ye, and preferably substantially all of the HFO-1225ye being fed to the reaction step A (it being recognized that the feed stream 1B in many embodiments will have a substantially zero flow and that in other embodiments this feed stream 1B may in fact be a recycle stream from subsequent operations in the process). The feed stream 2 comprises the hydrogenation or reducing agent, preferably H₂, for the reaction step A. Flow path or stream 4 is at path for allowing introduction of a recycle stream into the reaction step. In some embodiments, the actual flow of recycle stream 4 is zero, but in preferred embodiments, the recycle stream comprises a relatively low temperature stream comprising a portion of the reaction product stream 3A after it has been cooled and/or separated, the content of recycle stream 4, when present, preferably being relatively rich in HFC-236ea, HFC-245eb, or a combination of these.

In another preferred embodiment of the present invention as illustrated in Figure 3, the hydrogenation step comprises at least a first reaction step A1 and a second reaction step A2. In one embodiment, the first reaction step A1, which may comprise one or more reaction stages in parallel or in series or a combination of parallel or series, having associated therewith at least a first flow path or feed stream 1A and at least a second flow path or feed stream 2A, with each flow path being independently operable. In such embodiments, it is preferred that the first flow path or feed stream 1A comprises HFP and preferably substantially all of the HFP being fed to the reaction step A, the second path or feed stream 2A comprises the hydrogenation agent, preferably H₂, for the reaction step A. Flow path or stream 4A is at path for allowing introduction of a recycle stream into the reaction step. In some embodiments, the actual flow of recycle stream 4A is substantially zero, but in preferred embodiments, the recycle stream comprises a relatively low temperature stream comprising a portion of the reaction product stream 3A after it has been cooled and/or separated, the content of recycle stream 4, when present, preferably being relatively rich in HFC-236ea, HFC-245eb, or a combination of these.

In connection with the reaction stage converting HFP in the hydrogenation reactor at step A1, it is preferred in certain embodiments to use a trickle bed reactor. It is contemplated that the reaction is such case proceeds as follows:

CF₃CF=CF₂ + H₂ → CF₃CHFCF₂H (HFC-236ea).

A major side reaction of this process yields HFC-245eb and HF. It is believed that 245eb is formed from 236ea by hydrodefluorination and/or by dehydrofluorination followed by reduction:

HFC-236ea + H₂ → HFC-245eb + HF.

The second reaction step A2, may comprise one or more reaction stages in parallel or in series or a combination of parallel or series, having associated therewith at least a first flow path or feed stream 1B and at least a second flow path or feed stream 2B, with each flow path being independently operable. In such embodiments, it is preferred that the first flow path or feed stream 1B, when present, comprises HFO-1225ye, and preferably substantially all of the HFO-1225ye being fed to the reaction step A. The second path or feed stream 2B comprises the hydrogenation agent, preferably H₂, for the reaction step A2. Flow path or stream 4B is at path for allowing introduction of a recycle stream into the reaction step. In some embodiments, the actual flow of recycle stream 4B is zero, but in preferred embodiments, the recycle stream comprises a relatively low temperature stream comprising a portion of the reaction product stream 3A and/or 3B after it has been cooled and separated, the content of recycle stream 4B, when present, preferably being relatively rich in HFC-236ea, HFC-245eb, or a combination of these. Flow path or stream 10 is at path for allowing introduction of a second recycle stream into the reaction step, which in preferred embodiments comprises at least a portion of the reaction product stream 6 after being processed to comprise a stream relatively rich in HFO-1225ye.

In connection with the reaction stage converting HFO-1225ye in the hydrogenation reactor at step A2, it is preferred in certain embodiments to use a trickle bed reactor. It is contemplated that the reaction is such case proceeds as follows:

CF₃CF=CFH (liq) + H₂ (gas) → CF₃CHFCFH₂ (HFC-245eb - gas)

A major side reaction is contemplated to be:

HFC-245eb + H₂ → CF₃CHFCH₃ (HFC-254) + HF

Preferably, the hydrogenation reaction conditions are controlled in the reaction in order to achieve the desired conversion and/or selectivity in accordance with the present invention. As used herein, the term "reaction conditions" is intended to include the singular and means control of any one or more processing parameters, including possibly using or not using a reaction vessel or stage, which can be modified by the operator of the reaction to produce the conversion and/or selectivity of the feed material in accordance with the teachings contained herein. By way of example, but not by way of limitation, conversion of the feed material may be controlled or regulated by controlling or regulating any one or more of the following: the temperature of the reaction, the flow rate of the reactants, the presence of diluent, the amount of catalyst present in the reaction vessel, the shape and size of the reaction vessel, the pressure of the reaction, and any one combinations of these and other process parameters which will be available and known to those skilled in the art in view of the disclosure contained herein. The size and shape, and other characteristics of the reaction vessel itself may vary widely with the scope of the present invention, and it is contemplated that the vessel associated with each stage may be different than or the same as the vessel associated with the upstream and downstream reaction stages. Furthermore, it is contemplated that all reaction stages can occur inside a single vessel, provided that means and mechanisms necessary to control conversion are provided. For example, it may be desirable in certain embodiments to utilize a single tubular reactor for each reaction stage, providing conversion control by judicious selection of the amount and/or distribution of catalyst throughout the tubular reactor. In such a case, it is possible to further control the conversion in different sections of the same tubular reactor by controlling the amount of heat removed from or added to different sections of the tubular reactor.

Those skilled in the art will be readily able to select the type of catalyst(s) used for the hydrogenation step of the present invention in view of the teachings contained herein. For example, it is preferred in certain embodiments that at least one, but preferably all, reaction stages utilize palladium catalyst, preferably 1% palladium on carbon, either alone or in combination with other catalysts. In this regard one or more of the hydrogenation catalysts disclosed in US Patent 5, 679,875 may be used for one or more of the reaction stages in accordance with the present invention. In certain preferred embodiments, the catalyst preferably comprises palladium supported on carbon, such as a carbon mesh.

Thus, certain embodiments of the present methods comprise bringing a fluorinated olefin and a hydrogenation agent, such as H₂, into contact with a first amount of catalyst in at least a first reaction stage to produce a reaction stream comprising hydrofluorocarbon(s), unreacted fluorinated olefin and hydrogen. In certain preferred embodiments the hydrogenation step is followed by a preferred separation step as described below. While it is contemplated that a wide variety of hydrogenation reaction temperatures may be used, depending on relevant factors such as the catalyst being used and the most desired reaction product, it is generally preferred that the reaction temperature for the hydrogenation step is from about 50°C to about 150°C, preferably about from 75°C to about 115°C, and even more preferably from about 90°C to about 100°C.

In general, it is also contemplated that a wide variety of reaction pressures may be used, depending again on relevant factors such as the specific catalyst being used and the most desired reaction product. The reaction pressure can be, for example, from about 100 psig to about 300 psig (about 0.7 to about 2 Mpa gauge), preferably about from 150 psig to about 250 psig (about 1.0 to about 1.7 MPa gauge), and even more preferably about 200 psig (about 1 MPa gauge).

Applicants have found, without being bound by or to any particular theory, that the use of a cooled recycle stream 4, 4A, or 4B in the hydrogenation reaction allows the feed materials to serve as a means for removing heat from the hydrogenation reaction. Since the reduction or hydrogenation reaction of the present invention is generally exothermic, and usually substantially exothermic, the use of such a recycle material has the effect in preferred embodiments of maintaining the reactor temperature below that which would exist if the recycle were not used, assuming all other process conditions were maintained the same.

It is contemplated that the amount of hydrogen used may vary widely. In preferred embodiments, the hydrogen is feed to the reaction step as a gas in a H₂:olefin feed ratio of from about 1:1 to about 2: 1, and even more preferably ratio of from about 1:1 to about 1.5:1, and even more preferably about 1.3:1.

### THE HYDROGENATION REACTION EFFLUENT SEPARATION

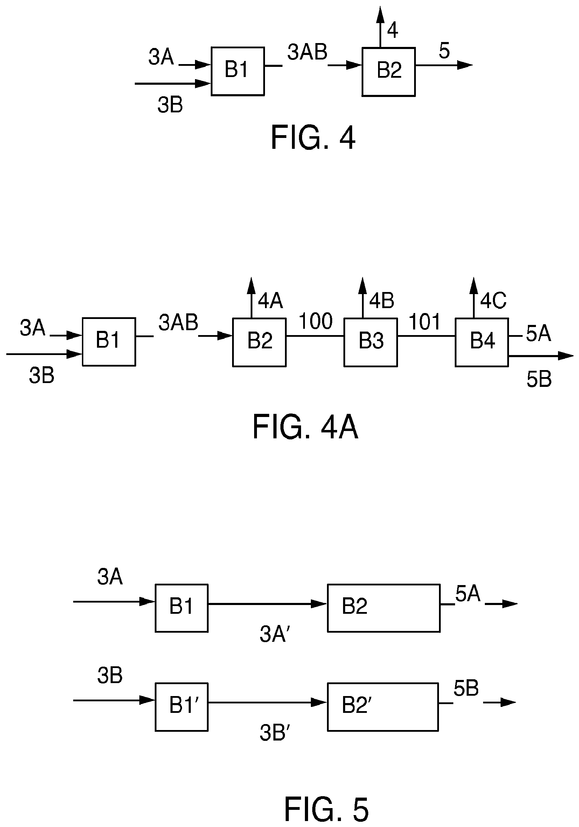

In certain preferred embodiments, the present invention also includes the step of cooling at least a portion reactor product stream (3, 3A, 3B) to remove at least a portion of the heat of reaction. In many preferred embodiments, this cooling step is included as part of the preferred aspects of the separation step B, which are described in connection with Figures 4, 5 and 6 below. Preferably the ratio of cooled recycled reaction product to fresh feed is about 12:1, with the temperature of the recycle stream preferably being at about 50 °C to about 100 °C, and even more preferably about 70 °C. In addition, in order to help remove heat of reaction, it is preferred in certain embodiments to introduce the fresh feeds and/or the recycle feeds to the reaction in the liquid phase and allowing the heat of reaction to evaporate the liquid feed and/or the reaction products and withdrawing the reaction products in the gas phase.

With reference now to Figure 4, the reaction product streams 3A and 3B are directed to a separation step B, which comprises in the embodiment of Figure 4 a cooling step B1 which produces one or more cooled reaction product streams 3AB, which in turn are fed to one or more separation stages B2. It is contemplated that those skilled in the art will be able to devise without undue experimentation many means and mechanisms for attaining such cooling in view of the teachings contained herein and all such means and mechanisms are with the scope of the present invention. The preferred separation step B2 preferably includes at least a first separation step which produces a first stream 4 relatively rich in unreacted hydrogen, fluorinated alkane, such as HFC-236ea and/or HFC-245eb, or a combination of these, which may be recycled, with or without further processing, to the reaction step A. A second stream 5, which is relatively rich in the fluorinated alkane, such as HFC-236ea and/or HFC-245eb, is also produced from the separation step B2.

In one preferred embodiment shown in Figure 4A, the separation step comprises, in addition to the cooling step B1 and the separation step B2 which produces at least a first cooled stream 4A containing a portion of the reaction product, which is preferably recycled to the reaction step A, and a crude product stream 100 which is fed to a further separation step B3 in which a substantial portion of excess hydrogen in the stream 100 is purged from the stream and sent for disposal or further processing in stream 4B. The stream 101 from the separation step B3 is then feed to a further separation step B4 where unwanted by-products are removed in stream 4C and one or more product streams 5A and 5B are produced. In preferred embodiments, stream 5A is relatively rich in a first fluorinated alkane, preferably HFC- 236ea, and a second stream 5B rich in a second fluorinated alkane, preferably HFC-245eb.

In another embodiment described with reference now to Figure 5, the reaction product streams 3A and 3B are each directed to a separate separation steps, which comprise separate cooling steps B1 and B1', each of which produces one or more cooled reaction product streams 3A' and 3B', which in turn are fed to separate separation stages B2 and B2' to produce a first stream 5A relatively rich in a first of the fluorinated alkane products, such as HFC-236ea when the feed stream 3A is rich in HFP, and a second reaction product stream 5B relatively rich in a second of the fluorinated alkane products, such as HFC-245eb when the feed stream 3B is rich in HFO-1225ye. A stream 4 (not shown) as described above in connection with Figure 4 may also be removed from each of the steps B2 and B2'. In addition, the particular embodiments shown and described in Figure 4A may also be adapted for use in connection with one or both the separation steps B and B' shown in Figure 5.

### DEHYDROHALOGENATION

The dehydrofluorination step can be carried out in a liquid phase in the presence of a dehydrohalogenation agent (e.g. caustic solution) or a gas phase in the presence of a dehydrofluorination catalyst. It is contemplated that the reaction may be carried out batchwise, continuously or a combination thereof.

In one embodiment the converting step involves a reaction in which HFC-245eb and/or HFC-236ea is contacted with a dehydrohalogenating agent which is KOH, wherein additional dehydrohalogenating agents can include, for example, NaOH, Ca(OH)₂, LiOH, Mg(OH)₂, CaO, and combinations thereof to form the fluorinated olefin. By way of example, as KOH is used, such a reaction may be described by way of illustration, but not necessarily by way of limitation, by the following reaction equations (1) and (2):

CF₃-CHF-CHF₂ + KOH → CF₃CF=CHF + KF + H₂O (1)

CF₃-CHF-CH₂F + KOH → CF₃CF=CH₂ + KF + H₂O (2)

The dehydrohalogenating agent may be provided as a caustic aqueous solution comprising from about 2% to about 100%, more preferably from about 10% to about 50%, and even more preferably from about 10% to about 30% by weight of dehydrohalogenating agent. In further embodiments, the caustic solution, and preferably the dehydrohalogenating agent solution, is brought to a temperature of from about 20°C to about 100°C, more preferably from about 20°C to about 90°C, and most preferably from about 20°C to about 70°C. The reaction pressure in such embodiments may vary, depending on particular processing parameters of each application. In certain embodiments, the reaction pressure ranges from atmospheric pressure, super-atmospheric pressure or under vacuum. The vacuum pressure, when used, in certain embodiments ranges from about 5 torr to about 760 torr (about 0.7 kPa to about 100 kPa).

It is contemplated that the amount of dehydrohalogenation agent (or reagent) used, or mole ratio of reagent to organic, will vary depending on the particular parameters present in each embodiment. In certain embodiments, the mol ratio of dehydrohalogenating agent to organic feeds (e.g. HFC-245eb and/or HFC-236ea) is from less than 1 to 3, preferably 1-1.5. In further embodiments, the contact time, which is expressed as the ratio of the volume of the reagent (ml) to the total feed flow (ml/sec) is from about 0.1 seconds to about 1000 seconds, and preferably from about 2 seconds to about 120 seconds.

The dehydrohalogenation reactions can be accomplished using any suitable vessel or reactor. Such vessels or reactors should be constructed from materials which are resistant to corrosion, such as stainless steel, nickel and its alloys, including Hastelloy, Inconel, Incoloy, and Monel. In certain embodiments, this reaction is performed using one or a series of Continuously Stirred Tank Reactors (CSTR). In this type of reactor, organic feed (e.g. HFC-236ea and/or HFC-245eb) and dehydrohalogenating agent would be fed continuously into the reactor and the resulting product stream formed would be fed into a condenser or distillation column for separation of 1225ye and/or 1234yf from and un-reacted 236ea and/or 245eb, as well as other by-products of the reaction.

In certain embodiments, spent dehydrohalogenating agent is removed from the product stream either periodically or continuously and is recycled back to the reactor for reuse. As noted previously, Applicants have discovered that, during continuous processing the reaction proceeds until either the organic reactants (e.g. HFC-236ea and/or HFC-245eb) or dehydrohalogenating agent is consumed. This increases costs of productivity because the reactor must be dismantled upon completion of the reaction to remove the salt and/or salt solutions. By recycling the dehydrohalogenating agent and by-product salts, however, such costs may be reduced and the system made more efficient.

Spent dehydrohalogenating agent and by-product salts (e.g. metal fluoride salts) may be withdrawn from the reactor by a product stream either continuously or intermittently using one or more known separation techniques. To this end, spent dehydrohalogenating agent separation may occur using any known compound separation techniques, such, but not limited to, distillation, phase separation, etc. In certain embodiments, withdrawal of spent dehydrohalogenating agent is especially beneficial for component separation as it allows for facile separation of organic and dehydrohalogenating agent. This in turn leads to lower cost associated with design and operation of complicated and highly specialized separation equipment.

The product stream containing spent dehydrohalogenating agent typically carries with it some dissolved organic (e.g. HFC-236ea and/or HFC-245eb). By stopping the stirrer and then removing spent dehydrohalogenating agent during the time agitation is stopped, separation of dehydrohalogenating agent and such organic can be facilitated. Spent dehydrohalogenating agent and dissolved organic would be taken into a container where additional separation of dehydrohalogenating agent and organic can be accomplished using one or more of the foregoing separation techniques. In one non-limiting embodiment, for example, KOH is separation by distillation, i.e. by heating organic just above boiling point of 236ea and/or 245eb, thus fractionating the organic from the spent KOH. Alternatively, one can use phase separator to separate between the two phases. The organic free KOH isolate can be immediately recycled to the reactor or can be concentrated and the concentrated solution can be returned to reactor.

The by-product salt can also be isolated and converted back to the dehydrohalogenating agent used known methods. When KOH is used as the dehydrohalogenating agent, KF is formed as a by-product salt. Such salt may be converted back to KOH and recycled back to the dehydrohalogenation reaction. For example, Ca(OH)₂ can be used for KF conversion according to reaction below.

2KF + Ca(OH)₂ → 2KOH + CaF₂

CaF₂ will precipitate from the foregoing reaction while KOH is isolated and recycled back to reactor. Recycling of spent dehydrohalogenating agent leads to better efficiency of the reagent use. Moreover, the use of recycling of the by-product salt reduces dehydrohalogenating agent use, reduces costs of reagents and costs associated with disposal of the salt, and/or purchase of new raw material.

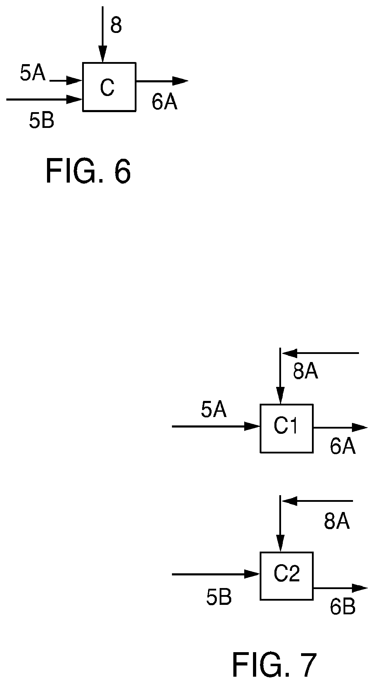

In one embodiment of the present invention, and referring to Figure 6, the dehydrohalogenation step comprises a reaction step C having associated therewith at least a first flow path or feed stream 5A, at least a second flow path or feed stream 5B and at least a third flow path or feed stream 8, with each flow path being independently operable. In such embodiments, it is preferred that the first flow path or feed stream 5A comprises HFO-236ea and preferably substantially all of the HFO-236ea being fed to the reaction step C, the second path or feed stream 5B comprises HFC-245eb, and preferably substantially all of the HFO-245eb being fed to the reaction step C (it being recognized that the feed stream 5B in many embodiments will have a substantially zero flow). Flow path or stream 8 is at path for allowing introduction of a recycle stream into the reaction step. In some embodiments, the actual flow of recycle stream 8 is zero, but in preferred embodiments, the recycle stream comprises a relatively low temperature stream comprising a portion of the reaction product stream 6A after it has been cooled and/or separated. The content of recycle stream 8, when present, may include unreacted organic (e.g. HFC-236ea, HFC-245eb) and spent dehydrohalogenating agent. Each may be purified or recovered, as provided herein and recycled.

In another preferred embodiment of the present invention as illustrated in Figure 7, the dehydrohalogenation step comprises at least a first reaction step C1 and a second reaction step C2. The first reaction step C1, which may comprise one or more reaction stages in parallel or in series or a combination of parallel or series, having associated therewith at least a first flow path or feed stream 5A and at least a second flow path or feed stream 8A, with each flow path being independently operable. The second reaction step C2, which may comprise one or more reaction stages in parallel or in series or a combination of parallel or series, having associated therewith at least a first flow path or feed stream 5B and at least a second flow path or feed stream 8B, with each flow path being independently operable. In such embodiments, it is preferred that the first flow path or feed stream 5A comprises HFO-236ea and preferably substantially all of the HFO-236ea being fed to the reaction step C, the second path or feed stream 5B comprises, when present, HFO-245eb and preferably substantially all of the HFO-245eb being fed to the reaction step C. Flow path or streams 8A and 8B are flow paths for allowing introduction of a recycle stream comprising at least a portion of unreacted feed back or recycled dehydrohalogenating agent, as defined herein. In some embodiments, the actual flow of recycle streams 8A and 8B are substantially zero, but in preferred embodiments, the recycle streams comprises relatively low temperature streams comprising a portion of the reaction product streams 6A and 6B after they have been cooled and separated.

Preferably, the reaction conditions are controlled in the reaction in order to achieve the desired conversion and/or selectivity in accordance with the present invention. As used herein, the term "reaction conditions" is intended to include the singular and means control of any one or more processing parameters, including possibly using or not using a reaction vessel or stage, which can be modified by the operator of the reaction to produce the conversion and/or selectivity of the feed material in accordance with the teachings contained herein. By way of example, but not by way of limitation, conversion of the feed material may be controlled or regulated by controlling or regulating any one or more of the following: the temperature of the reaction, the flow rate of the reactants, the presence of diluent, the amount of catalyst present in the reaction vessel, the shape and size of the reaction vessel, the pressure of the reaction, and any one combinations of these and other process parameters which will be available and known to those skilled in the art in view of the disclosure contained herein. The size and shape, and other characteristics of the reaction vessel itself may vary widely with the scope of the present invention, and it is contemplated that the vessel associated with each stage may be different than or the same as the vessel associated with the upstream and downstream reaction stages. Furthermore, it is contemplated that all reaction stages can occur inside a single vessel, provided that means and mechanisms necessary to control conversion are provided. For example, it may be desirable in certain embodiments to utilize a single tubular reactor for each reaction stage, providing conversion control by judicious selection of the amount and/or distribution of catalyst throughout the tubular reactor. In such a case, it is possible to further control the conversion in different sections of the same tubular reactor by controlling the amount of heat removed from or added to different sections of the tubular reactor.

The resulting products may be isolated from the product stream using one or more methods known in the art and purified accordingly.

### EXAMPLES

The following examples are provided for the purpose of illustrating the present invention but without limiting the scope thereof.

### Example 1:

A CSTR (Continuously Stirred Tank Reactor) was used to convert 245eb to 1234yf. The reactor was designed and operated in such a way that the end of the dip tube was in the middle of KOH phase. After the startup, organic and fresh KOH were constantly fed in. Organic out (the overhead portion) was constantly taken out. In a typical cycle organic and KOH flow in and overhead is taken out for 30 min. Flowrates were 5 and 12 ml/min, respectively. The mixture is stirred during this period of time. Then stirrer is stopped for 10 min, while feeds still go in. While stirrer is off, after 10 min, applicable valves for the spent KOH to go out were open. Between 500 and 550 g was taken out every 40 min.

Experiment was carried out for 7 hours. Temperature was 48 °C. Pressure was 45 psig (310 kPa gauge). GC area percent of the organic feed was ~97% of 245eb, the rest being most probably 254. The flowrates translate to mol ratio of KOH to G-245eb of 1, or slightly above 1. Theoretical residence time was 100 min.

Scrubber Product Collection Cylinder (SPCC) was used to collect spent KOH and the portion of organic that was carried out with spent KOH. Organic was trapped in methylene chloride which was previously added to SPCC. The volume of methylene chloride inside the SPCC was 15% of the total volume of liquid in SPCC. Product and un-reacted organic was collected in a Product Collection Cylinder (PCC) that was held in dry ice. Vapor samples were taken once per hour while liquid samples were taken at much longer intervals.

Overall mass balance was 94% for organic. Average conversion was 48%. Selectivity to 1234yf was 73%.

### Example 2:

The second experiment was carried out in the same fashion as the experiment 1, except that pressure and temperature were 48 psig (330 kPa gauge) and 47 °C. Experiment lasted 6 hours and overall conversion was 69%. Selectivity to 1234yf was 75%.

### Example 3:

Reactor was loaded with 807 ml of organic. The feed was analyzed by GC to reveal Area % for 236ea as 97.70%. There was no 1234yf in this feed, while the percentage of 1225ye was 0.03. After loading the reactor with organic, 1641 ml of 25% KOH was added. Therefore mol ratio of KOH to 236ea was 1.23.

The flowrates of 236ea and KOH were 2 and 4 ml/min. Pressure was 35-45 psig (240-310 kPa gauge). Reactor temperature was 42-45 °C.

Spent KOH was taken out of reactor periodically (it was fed continuously as was organic; overhead product was taken out continuously, i.e. semi-continuous operation). In this semi-continuous mode KOH and organics flowed into stirred reactor for 50 min. Then the stirrer was stopped for 10 min while reactants were still going in. Afterwards 290-300 g of spent KOH was taken out. Then stirrer was started as was the clock for 50 min time period.

Spent KOH was collected in Scrubber Product Collection Cylinder (SPCC). SPCC was pre-filled with methylene chloride (the target was to have 15-20 wt% of methylene chloride in the final spent KOH- methylene chloride solution). Analysis of methylene chloride layer revealed that ~70 GC area % was 236ea. There was small amount of 1225ye (~2%) while the rest were unidentified heavies. In order to confirm that all organic was extracted with methylene chloride , part KOH was added to part MeCl₂ (i.e. 1:1). GC analysis revealed that amount of organic was negligible.

Overall mass balance was 88.67%. Residence time was 200 min. The conversion was ~50%. Selectivity to 1225ye was 63%. The reaction was carried out for the total of 97 hours.

## Claims

1. A method for producing at least one fluorinated olefin comprising:
a. hydrogenating a starting material stream comprising at least one alkene comprising hexafluoropropylene by contacting said starting material with a reducing agent to produce an intermediate product stream comprising at least one alkane comprising 1,1,1,2,3,3-hexafluoropropane and 1,1,1,2,3-pentafluoropropane;
b. optionally, separating said intermediate product stream into a plurality of intermediate product streams, said plurality of intermediate product streams comprising two or more streams selected from the group consisting of a first stream rich in 1,1,1,2,3,3-hexafluoropropane, a second stream rich in 1,1,1,2,3-pentafluoropropane, and an alkane recycle stream;
c. dehydrofluorinating, in the presence of a dehydrohalogenating agent, at least a portion of said intermediate process stream from step (a) or said plurality of intermediate process streams of step (b) to produce an alkene product stream comprising 1,1,1,2,3-pentafluoropropene and at least one additional alkene comprising 2,3,3,3-tetrafluoropropene, wherein the dehydrohalogenating agent is KOH; and
d. withdrawing from a dehydrofluorinated product stream a product reaction stream comprising spent KOH, dissolved organics and KF; and
e. recovering spent KOH, wherein recovering spent KOH comprises a step of separating the spent KOH and the KF in the product reaction stream from the dissolved organics; and
f. recycling the recovered spent KOH from step (e) back to the dehydrohalogenation reaction.

2. The method of claim 1, wherein step (b) is not present.

3. The method of claim 1, wherein step (b) is present.

4. The method of any preceding claim, wherein the reducing agent comprises H₂.

5. The method of any preceding claim further comprising converting the KF to KOH.

6. The method of claim 5 further comprising concentrating the converted KOH and recycling it back to the dehydrohalogenation reaction.

7. A method for producing 2,3,3,3-tetrafluoropropene comprising:
a. hydrogenating a starting material stream comprising hexafluoropropylene by contacting said starting material stream with a reducing agent in a hydrogenation reactor to produce an intermediate stream comprising 1,1,1,2,3-pentafluoropropane or 1,1,1,2,3,3-hexafluoropropane;
b. dehydrofluorinating, in the presence of a dehydrohalogenating agent, said 1,1,1,2,3-pentafluoropropane or said 1,1,1,2,3,3-hexafluoropropane in a dehydrofluorination reactor to produce a product stream comprising 2,3,3,3-tetrafluoropropene or 1,1,1,2,3-pentafluoropropene, wherein the dehydrohalogenating agent is KOH;
c. withdrawing from the product stream a second product stream comprising spent KOH, dissolved organics and KF;
d. recovering spent KOH, wherein recovering spent KOH comprises a step of separating the spent KOH and the KF in the second product stream from the dissolved organics; and
e. concentrating the purified KOH and recycling it back to the dehydrohalogenation reaction.

8. The method of claim 7 wherein the dissolved organics are selected from the group consisting of 1,1,1,2,3,3-hexafluoropropane (HFC-236ea) and/or 1,1,1,2,3-pentafluoropropane (HFC-245eb).

9. The method of claim 7 or claim 8 further comprising converting the KF to KOH.

10. The method of claim 9 further comprising concentrating the converted KOH and recycling it back to the dehydrohalogenation reaction.

11. The method of any of claims 7 to 10, wherein the reducing agent comprises H₂.

12. A process for the manufacture of a fluoroolefin comprising:
(a) dehydrohalogenating 1,1,1,2,3,3-hexafluoropropane (HFC-236ea) or 1,1,1,2,3-pentafluoropropane (HFC-245eb) in the presence of KOH to produce 2,3,3,3-tetrafluoropropene (HFO-1234yf) or 1,2,3,3,3-pentafluoropropene (HFO-1225ye);
(b) withdrawing a reaction stream comprising spent KOH, dissolved organics, and KF;
(c) recovering spent KOH, wherein recovering spent KOH comprises a step of separating the spent KOH and the KF in the reaction stream from the dissolved organics;
(d) optionally converting KF to KOH; and
(e) recycling the recovered spent KOH from step (c) back to the dehydrohalogenation reaction.

13. The process of claim 12 further comprising, optionally, concentrating the converted KOH and recycling it back to the dehydrohalogenation reaction.

14. The method of claim 12 or claim 13, wherein step (d) is not present.

15. The method of claim 12 or claim 13, wherein step (d) is present.

## Patentansprüche

1. Verfahren zur Herstellung mindestens eines fluorierten Olefins, umfassend:
a. Hydrieren eines Ausgangsmaterialstroms, umfassend mindestens ein Alken, das Hexafluorpropylen umfasst, durch Inkontaktbringen des Ausgangsmaterials mit einem Reduktionsmittel, um einen Zwischenproduktstrom herzustellen, der mindestens ein Alkan umfasst, das 1,1,1,2,3,3-Hexafluorpropan und 1,1,1,2,3-Pentafluorpropan umfasst;
b. optional Trennen des Zwischenproduktstroms in eine Vielzahl von Zwischenproduktströmen, wobei die Vielzahl von Zwischenproduktströmen zwei oder mehr Ströme umfasst, die ausgewählt sind aus der Gruppe, bestehend aus einem ersten Strom, der reich an 1,1,1,2,3,3-Hexafluorpropan ist, einem zweiten Strom, der reich an 1,1,1,2,3-Pentafluorpropan ist, und einem Alkan-Recyclingstrom;
c. Dehydrofluorieren mindestens eines Teils des Zwischenprozessstroms aus Schritt (a) oder der Vielzahl von Zwischenprozessströmen aus Schritt (b) in Gegenwart eines Dehydrohalogenierungsmittels, um einen Alken-Produktstrom herzustellen, der 1,1,1,2,3-Pentafluorpropen und mindestens ein zusätzliches Alken umfasst, das 2,3,3,3-Tetrafluorpropen umfasst, wobei es sich bei dem Dehydrohalogenierungsmittel um KOH handelt; und
d. Entnehmen eines Produktreaktionsstroms, umfassend verbrauchtes KOH, gelöste organische Stoffe und KF, aus einem dehydrofluorierten Produktstrom; und
e. Rückgewinnen von verbrauchtem KOH, wobei das Rückgewinnen von verbrauchtem KOH einen Schritt des Trennens des verbrauchten KOH und des KF im Produktreaktionsstrom von den gelösten organischen Stoffen umfasst; und
f. Recyceln des zurückgewonnenen verbrauchten KOH aus Schritt (e) zurück in die Dehydrohalogenierungsreaktion.

2. Verfahren nach Anspruch 1, wobei Schritt (b) nicht vorhanden ist.

3. Verfahren nach Anspruch 1, wobei Schritt (b) vorhanden ist.

4. Verfahren nach einem der vorhergehenden Ansprüche, wobei das Reduktionsmittel H₂ umfasst.

5. Verfahren nach einem der vorhergehenden Ansprüche, ferner umfassend das Umwandeln des KF in KOH.

6. Verfahren nach Anspruch 5, ferner umfassend das Konzentrieren des umgewandelten KOH und dessen Recycling zurück in die Dehydrohalogenierungsreaktion.

7. Verfahren zur Herstellung von 2,3,3,3-Tetrafluorpropen, umfassend:
a. Hydrieren eines Ausgangsmaterialstroms, umfassend Hexafluorpropylen, durch Inkontaktbringen des Ausgangsmaterialstroms mit einem Reduktionsmittel in einem Hydrierungsreaktor, um einen Zwischenstrom herzustellen, der 1,1,1,2,3-Pentafluorpropan oder 1,1,1,2,3,3-Hexafluorpropan umfasst;
b. Dehydrofluorieren des 1,1,1,2,3-Pentafluorpropans oder des 1,1,1,2,3,3-Hexafluorpropans in einem Dehydrofluorierungsreaktor in Gegenwart eines Dehydrohalogenierungsmittels, um einen Produktstrom herzustellen, der 2,3,3,3-Tetrafluorpropen oder 1,1,1,2,3-Pentafluorpropen umfasst, wobei es sich bei dem Dehydrohalogenierungsmittel um KOH handelt;
c. Entnehmen eines zweiten Produktstroms, umfassend verbrauchtes KOH, gelöste organische Stoffe und KF, aus dem Produktstrom;
d. Rückgewinnen von verbrauchtem KOH, wobei das Rückgewinnen von verbrauchtem KOH einen Schritt des Trennens des verbrauchten KOH und des KF in dem zweiten Produktstrom von den gelösten organischen Stoffen umfasst; und
e. Konzentrieren des gereinigten KOH und dessen Recycling zurück in die Dehydrohalogenierungsreaktion.

8. Verfahren nach Anspruch 7, wobei die gelösten organischen Stoffe ausgewählt sind aus der Gruppe, bestehend aus 1,1,1,2,3,3-Hexafluorpropan (HFC-236ea) und/oder 1,1,1,2,3-Pentafluorpropan (HFC-245eb).

9. Verfahren nach Anspruch 7 oder Anspruch 8, ferner umfassend das Umwandeln des KF in KOH.

10. Verfahren nach Anspruch 9, ferner umfassend das Konzentrieren des umgewandelten KOH und dessen Recycling zurück in die Dehydrohalogenierungsreaktion.

11. Verfahren nach einem der Ansprüche 7 bis 10, wobei das Reduktionsmittel H₂ umfasst.

12. Prozess zur Herstellung eines Fluorolefins, umfassend:
(a) Dehydrohalogenieren von 1,1,1,2,3,3-Hexafluorpropan (HFC-236ea) oder 1,1,1,2,3-Pentafluorpropan (HFC-245eb) in Gegenwart von KOH, um 2,3,3,3-Tetrafluorpropen (HFO-1234yf) oder 1,2,3,3,3-Pentafluorpropen (HFO-1225ye) herzustellen;
(b) Entnehmen eines Reaktionsstroms, umfassend verbrauchtes KOH, gelöste organische Stoffe und KF;
(c) Rückgewinnen des verbrauchten KOH, wobei das Rückgewinnen des verbrauchten KOH einen Schritt des Trennens des verbrauchten KOH und des KF im Reaktionsstrom von den gelösten organischen Stoffen umfasst;
(d) optional Umwandeln von KF in KOH; und
(e) Recyceln des zurückgewonnenen verbrauchten KOH aus Schritt (c) zurück in die Dehydrohalogenierungsreaktion.

13. Prozess nach Anspruch 12, ferner optional umfassend das Konzentrieren des umgewandelten KOH und dessen Recycling zurück in die Dehydrohalogenierungsreaktion.

14. Verfahren nach Anspruch 12 oder Anspruch 13, wobei Schritt (d) nicht vorhanden ist.

15. Verfahren nach Anspruch 12 oder Anspruch 13, wobei Schritt (d) vorhanden ist.

## Revendications

1. Procédé de production d'au moins une oléfine fluorée comprenant :
a. l'hydrogénation d'un flux de matière première comprenant au moins un alcène comprenant de l'hexafluoropropylène par la mise en contact de ladite matière première avec un agent réducteur pour produire un flux de produit intermédiaire comprenant au moins un alcane comprenant du 1,1,1,2,3,3-hexafluoropropane et du 1,1,1,2,3-pentafluoropropane ;
b. la séparation éventuelle dudit flux de produit intermédiaire en une pluralité de flux de produits intermédiaires, ladite pluralité de flux de produits intermédiaires comprenant deux flux ou plus choisis dans le groupe constitué d'un premier flux riche en 1,1,1,2,3,3-hexafluoropropane, d'un deuxième flux riche en 1,1,1,2,3-pentafluoropropane et d'un flux de recyclage d'alcane ;
c. la déshydrofluoration, en présence d'un agent de déshydrohalogénation, d'au moins une partie dudit flux de traitement intermédiaire de l'étape (a) ou ladite pluralité de flux de traitement intermédiaire de l'étape (b) pour produire un flux de produit alcène comprenant le 1,1,1,2,3-pentafluoropropène et au moins un alcène supplémentaire comprenant le 2,3,3,3-tétrafluoropropène, dans lequel l'agent de déshydrohalogénation est le KOH ; et
d. le prélèvement, à partir d'un flux de produit déshydrofluoré, d'un flux de réaction de produit comprenant du KOH usé, des matières organiques dissoutes et du KF ; et
e. la récupération du KOH usé, dans lequel la récupération du KOH usé comprend une étape de séparation du KOH usé et du KF dans le flux de produit de réaction à partir des matières organiques dissoutes ; et
f. le recyclage du KOH usé récupéré de l'étape (e) dans la réaction de déshydrohalogénation.

2. Procédé selon la revendication 1, dans lequel l'étape (b) n'est pas présente.

3. Procédé selon la revendication 1, dans lequel l'étape (b) est présente.

4. Procédé selon l'une quelconque des revendications précédentes, dans lequel l'agent réducteur comprend du H₂.

5. Procédé selon l'une quelconque des revendications précédentes, comprenant en outre la conversion de KF en KOH.

6. Procédé selon la revendication 5, comprenant en outre la concentration du KOH converti et son recyclage dans la réaction de déshydrohalogénation.

7. Procédé de production de 2,3,3,3-tétrafluoropropène comprenant :
a. l'hydrogénation d'un flux de matière première comprenant de l'hexafluoropropylène par la mise en contact dudit flux de matière première avec un agent réducteur dans un réacteur d'hydrogénation pour produire un flux intermédiaire comprenant du 1,1,1,2,3-pentafluoropropane ou du 1,1,1,2,3,3-hexafluoropropane ;
b. la déshydrofluoration, en présence d'un agent de déshydrohalogénation, dudit 1,1,1,2,3-pentafluoropropane ou dudit 1,1,1,2,3,3-hexafluoropropane dans un réacteur de déshydrofluoration pour produire un flux de produit comprenant du 2,3,3,3-tétrafluoropropène ou du 1,1,1,2,3-pentafluoropropène, dans lequel l'agent de déshydrohalogénation est le KOH ;
c. le prélèvement, à partir du flux de produit, d'un deuxième flux de réaction de produit comprenant du KOH usé, des matières organiques dissoutes et du KF ;
d. la récupération du KOH usé, dans lequel la récupération du KOH usé comprend une étape de séparation du KOH usé et du KF dans le deuxième flux de produit à partir des matières organiques dissoutes ; et
e. la concentration du KOH purifié et son recyclage dans la réaction de déshydrohalogénation.

8. Procédé selon la revendication 7, dans lequel les matières organiques dissoutes sont choisies dans le groupe constitué du 1,1,1,2,3,3-hexafluoropropane (HFC-236ea) et/ou du 1,1,1,2,3-pentafluoropropane (HFC-245eb).

9. Procédé selon la revendication 7 ou la revendication 8, comprenant en outre la conversion de KF en KOH.

10. Procédé selon la revendication 9, comprenant en outre la concentration du KOH converti et son recyclage dans la réaction de déshydrohalogénation.

11. Procédé selon l'une quelconque des revendications 7 à 10, dans lequel l'agent réducteur comprend du H₂.

12. Procédé de fabrication d'une fluorooléfine comprenant :
(a) la déshydrohalogénation du 1,1,1,2,3,3-hexafluoropropane (HFC-236ea) ou du 1,1,1,2,3-pentafluoropropane (HFC-245eb) en présence de KOH pour produire du 2,3,3,3-tétrafluoropropène (HFO-1234yf) ou du 1,2,3,3,3-pentafluoropropène (HFO-1225ye) ;
(b) le prélèvement d'un flux de réaction comprenant du KOH usé, des matières organiques dissoutes et du KF ;
(c) la récupération du KOH usé, dans lequel la récupération du KOH usé comprend une étape de séparation du KOH usé et du KF dans le flux de réaction à partir des matières organiques dissoutes ;
(d) la conversion éventuelle du KF en KOH ; et
(e) le recyclage du KOH usé récupéré de l'étape (c) dans la réaction de déshydrohalogénation.

13. Procédé selon la revendication 12, comprenant en outre la concentration éventuelle du KOH converti et son recyclage dans la réaction de déshydrohalogénation.

14. Procédé selon la revendication 12 ou la revendication 13, dans lequel l'étape (d) n'est pas présente.

15. Procédé selon la revendication 12 ou la revendication 13, dans lequel l'étape (d) est présente.
